# EUROPEAN PATENT SPECIFICATION

(11) **EP 3 661 494 B1**
(45) Date of publication and mention of the grant of the patent: **11.10.2023**
(21) Application number: 18755664.2
(22) Date of filing: 02.08.2018
(51) Int. Cl.: A61K 31/343, A61K 45/06, A61P 25/24

(54) **USE OF TASIMELTEON FOR THE TREATMENT OF AFFECTIVE DISORDERS IN MAJORITY BLACK AFRICAN PATIENTS**
VERWENDUNG VON TASIMELTEON ZUR BEHANDLUNG VON AFFEKTIVEN STÖRUNGEN IN MEHRHEITLICH SCHWARZAFRIKANISCHE PATIENTEN
UTILISATION DE TASIMELTÉON POUR LE TRAITEMENT DE TROUBLES AFFECTIFS CEHZ PATIENTS EN MAJORITÉ NOIR AFRICAINS

(30) Priority: 02.08.2017 US 201762540200 P
(43) Date of publication of application: 10.06.2020
(73) Proprietor: Vanda Pharmaceuticals Inc., Washington, DC 20037 (US); Polymeropoulos, Mihael, Potomac, MD 20854 (US); Polymeropoulos, Christos, Potomac, MD 20854 (US); Xiao, Changfu, McLean, VA 22101 (US)
(72) Inventor: POLYMEROPOULOS, Mihael, Vienna VA 22182 (US); POLYMEROPOULOS, Christos, Vienna VA 22182 (US); XIAO, Changfu, McLean, VA 22101 (US)
(74) Representative: Appleyard Lees IP LLP
(86) International application number: PCT/US2018/044995
(87) International publication number: WO 2019/028245

(56) References cited:
- US-A1- 2009 209 638
- STEVEN W LOCKLEY ET AL: "Tasimelteon for non-24-hour sleep-wake disorder in totally blind people (SET and RESET): two multicentre, randomised, double-masked, placebo-controlled phase 3 trials", LANCET, vol. 386, no. 10005, 1 October 2015 (2015-10-01), pages 1754-1764, XP055385507, AMSTERDAM, NL ISSN: 0140-6736, DOI: 10.1016/S0140-6736(15)60031-9
- RAJARATNAM S M ET AL: "Melatonin agonist tasimelteon (VEC-162) for transient insomnia after sleep-time shift: two randomised controlled multicentre trials", LANCET, ELSEVIER, AMSTERDAM, NL, vol. 373, no. 9662, 7 February 2009 (2009-02-07), pages 482-491, XP025913508, ISSN: 0140-6736, DOI: 10.1016/S0140-6736(08)61812-7 [retrieved on 2009-02-05]
- POLYMEROPOULOS M H ET AL: "Tasimelteon improves symptoms of major depression in an African American population", SLEEP MEDICINE, vol. 40, 1 December 2017 (2017-12-01), XP085325052, ISSN: 1389-9457, DOI: 10.1016/J.SLEEP.2017.11.779
- HULL J T ET AL: "Differences in the timing of melatonin secretion between African, American and Caucasian patients with major depressive disorder", SLEEP MEDICINE, vol. 40, 1 December 2017 (2017-12-01), XP085324681, ISSN: 1389-9457, DOI: 10.1016/J.SLEEP.2017.11.407

## Description

### BACKGROUND

Affective disorders are mood disorders and include, among other disorders, depressive disorders. Illustrative affective disorders include attention deficit hyperactivity disorder (ADHD), bipolar disorder, body dysmorphic disorder, bulimia nervosa and other eating disorders, cataplexy, dysthymia, generalized anxiety disorder, hypersexuality, irritable bowel syndrome (IBS), impulse-control disorders, kleptomania, migraine, major depressive disorder (MDD), obsessive-compulsive disorder (OCD), oppositional defiant disorder, panic disorder, post-traumatic stress disorder (PTSD), premenstrual dysphoric disorder, social anxiety disorder, and fibromyalgia.

In addition, other disorders may be part of a spectrum accompanying or associated with affective disorders. These include, for example, chronic pain, intermittent explosive disorder, pathological gambling, personality disorder, pyromania, substance abuse and addiction including alcoholism, and trichotillomania.

Depressive disorders affect nearly 20 million adults in the US and are characterized by an array of symptoms, including persistent sad, anxious, or empty mood; feelings of hopelessness or pessimism; feelings of guilt, worthlessness, or helplessness; loss of interest or pleasure in hobbies and activities that were once enjoyed, including sex; decreased energy, fatigue, or a feeling of being "slowed down;" difficulty concentrating, remembering, or making decisions; insomnia, early-morning awakening, or oversleeping; appetite and/or weight loss; overeating and weight gain; thoughts of death or suicide; suicide attempts; restlessness or irritability; and persistent physical symptoms that do not respond to treatment, such as headaches, digestive disorders, and chronic pain. Untreated depressive disorders may be both emotionally and physically debilitating.

The National Institute of Mental Health (NIMH) has identified three of the most common types of depressive illness as: Major depressive disorder (MDD), dysthymia, and bipolar disorder (also referred to as manic-depressive illness).

MDD is manifested by a combination of symptoms that interfere with one's ability to work, study, sleep, eat, and enjoy once-pleasurable activities. MDD may occur only once, but more commonly occurs several times during a patient's lifetime. MDD is often associated with insomnia and it has been hypothesized that circadian rhythm disruption may be involved in the etiology of MDD.

Dysthymia is typically less severe than MDD and involves long-term, chronic symptoms that do not disable but prevent one from functioning well or feeling well. Patients with dysthymia may also experience episodes of MDD at times.

Bipolar disorder is not as common as other depressive disorders and is characterized by mood changes between severe highs (manic episodes) and severe lows (depressive episodes). Mood changes may be rapid and dramatic, but are more typically gradual. During a depressive episode, an individual may experience any or all of the symptoms of other depressive disorders. During a manic episode, an individual may be overactive, overly talkative, and have a lot of energy. Manic episodes often affect thinking, judgment, and social behavior that result in embarrassment or other problems. Untreated, manic episodes may worsen to a psychotic state.

Tasimelteon (*trans*-N-[[2-(2,3-dihydrobenzofuran-4-yl)cycloprop-1yl]methyl] propanamide) is a circadian regulator which binds specifically to two high affinity melatonin receptors, Mella (MT1R) and Mellb (MT2R). These receptors are found in high density in the suprachiasmatic nucleus of the brain (SCN), which is responsible for synchronizing our sleep/wake cycle.

Tasimelteon is disclosed in US Patent No. 5,856,529 and US Patent Application Publication No. 2009/0105333. Tasimelteon improves sleep parameters in clinical studies which simulate a desynchronization of the circadian clock. Tasimelteon has so far been studied in hundreds of individuals and is shown a good tolerability profile.

The use of tasimelteon in treating depressive disorders is described in US Patent Application Publication No. 2009/0209638.

The use of tasimelteon in entraining the circadian rhythm of an individual to a 24-hour period is described in US Patent No. 8,785,492.

The effects of CYP1A2 inhibitors and CYP3A4 inhibitors on the administration of tasimelteon are described in US Patent Application Publication No. 2015/0025086.

The effects of food on the bioavailablity of tasimelteon are described in US Patent Application Publication No. 2015/0196527.

Lockley S et al (Lancet, vol. 386, no. 10005, pages 1754-64, 31 October 2015) discloses the use of tasimelteon for the treatment of non-24-hour sleep-wake disorder in totally blind people.

Rajaratnam SM et al (Lancet, vol. 373, no. 9662, pages 482-491, 7 February 2009) discloses the use of tasimelteon (VEC-162) for the treatment of transient insomnia after sleep-time shift.

An individual with genetic "Majority Black African" ancestry, as used in this specification, refers to an individual having ancestry in any of the black African populations that is about 50% or greater black African ancestry. This definition encompasses individuals, for example, with about 50% or 70% or greater West African ancestry. African-Americans, as that term is used here, is defined as a subpopulation of Americans who are "Majority Black African." In this regard, Bryc *et al.,* for example, report that, on average, African-American populations comprise 73.2-82.1% West African, 16.7-24% European, and 0.8-1.2% Native American genetic ancestry, although there is large individual variation.

### SUMMARY

The present invention is defined by the appended claims. In the following description, where reference is made to the invention encompassing a method of treating a disease using a compound, we intend such references to be interpreted as relating to said compounds for use in said methods of treating said disease.

The invention relates generally to the treatment of an affective disorder in individuals of Majority Black African genetic ancestry, including African-Americans by administering to the patient an amount of tasimelteon effective to treat such disorder.

According to various embodiments of the invention, tasimelteon is administered: at a dose of 20 mg/day, prior to bedtime (*e*.*g*., 0.5 hour to 2 hours prior), under fasted conditions, while avoiding the coadministration of rifampicin, while avoiding the coadministration of fluvoxamine, while avoiding administration to a patient who smokes, to a patient having high or low endogenous melatonin, or any combination thereof.

In some embodiments of the invention, the treatment of an affective disorder in a Majority Black African (including African-American) patient includes entraining the patient's circadian rhythm, melatonin rhythm, and/or cortisol rhythm to a 24-hour sleep-wake cycle by administering to the patient an effective amount of tasimelteon.

### DETAILED DESCRIPTION

A large clinical study was conducted to evaluate the effects of tasimelteon on depressive symptoms in patients with MDD. Details of the study design are set out in study number NCT01428661, available at clinicaltrials.gov. Aspects of the study relevant to the claimed invention are described below.

A total of 507 patients with MDD were enrolled in a double-masked, placebo-controlled study randomized on either tasimelteon 20 mg (n=254) or placebo (n=253). Patients were assessed at baseline and weekly for eight weeks on a number of depression scales, including the Hamilton Depression Scale (HAMD).

Tasimelteon- and placebo-treated patients appeared to improve similarly from baseline by 8.1 and 7.8 points, respectively, on the HAMD scale (p=0.57). An analysis by reported race, however, reveals a significant positive effect of tasimelteon among African-American patients. Of the 507 randomized patients, 166 (32.7%) are African-American, of which 78 received tasimelteon and 88 placebo. A majority of the remaining patients included in the study are Caucasian.

At eight weeks, African-American MDD patients treated with tasimelteon show improvement by 9.9 points on the HAMD scale, as compared to 6.9 points for the placebo-treated patients (p=0.018). In a responder analysis (improvement in the HAMD scale of 50% or greater from baseline), 59% of tasimelteon-treated show improvement, as compared to 30% of placebo-treated patients (p=0.0019).

These results suggest that tasimelteon 20 mg improves symptoms of depression in African-American patients with MDD. These results also support the suggestion noted above of a circadian component in the etiology and treatment of MDD.

In addition, among the study subjects, melatonin onset (MO) occurs significantly (p=0.0229) earlier among African-American MDD patients than Caucasian MDD patients. In African-American MDD patients, mean MO occurs at 21:07 ± 1:53 hours (range 14:58-01:50 hours). In Caucasian MDD patients, mean MO occurs at 21:36 ± 1:53 hours (range 16:23-03:54 hours). This additional observation may help to explain the improved efficacy in African-Americans as compared to non-African-American participants in the study.

The invention therefore provides the treatment of an affective disorder in a Majority Black African patient in need of such treatment by administering to the patient an amount of tasimelteon effective to treat said affective disorder.

Such administration, according to some embodiments, is prior to bedtime, *e*.*g*., between about 0.5 hour and about 2 hours prior to a target bedtime.

According to some embodiments, the amount of tasimelteon effective to treat the affective disorder is between 10 mg/day and 100 mg/day, *e*.*g*., between 20 mg/day and 50 mg/day, *e*.*g*., 20 mg/day.

Other embodiments include entraining a circadian rhythm, a cortisol rhythm, or a melatonin rhythm of a Majority Black African patient to a 24-hour sleep-wake cycle by orally administering to the patient 20 mg of tasimelteon once daily before a target bedtime. In such embodiments, a 24-hour sleep-wake cycle is a cycle in which the patient goes to sleep at or near a target bedtime and awakens at or near a target wake time following a daily sleep period of approximately 7 to 9 hours.

These and other embodiments may include administering tasimelteon to a Majority Black African patient after discontinuing the administration of fluvoxamine or rifampicin to said patient, administering tasimelteon to such patient after such patient discontinues smoking, and/or administering tasimelteon to such patient without food.

## Claims

1. Tasimelteon for use in the treatment of an affective disorder in a Majority Black African patient, the treatment comprising:
administering to said patient an amount of tasimelteon effective to treat said affective disorder.

2. Tasimelteon for the use of claim 1, wherein the affective disorder is a depressive disorder.

3. Tasimelteon for the use of claim 1, wherein the patient exhibits at least one symptom selected from a group consisting of: persistent sad, anxious, or empty mood; feelings of hopelessness; pessimism; feelings of guilt, worthlessness, or helplessness; loss of interest or pleasure in hobbies and activities that were once enjoyed, including sex; decreased energy, fatigue, or being slowed down; difficulty concentrating, remembering, or making decisions; insomnia, early-morning awakening, or oversleeping; appetite and/or weight loss or overeating and weight gain; thoughts of death or suicide; suicide attempts; restlessness; irritability; persistent physical symptoms that do not respond to treatment, such as headaches, digestive disorders, and chronic pain; or any combination of the preceding.

4. Tasimelteon for the use of claim 1, wherein the affective disorder is major depressive disorder (MDD).

5. Tasimelteon for the use of claim 1, 2, 3, or 4, wherein the effective amount is between 10 mg/day and 100 mg/day.

6. Tasimelteon for the use of claim 5, wherein the effective amount is between 20 mg/day and 50 mg/day.

7. Tasimelteon for the use of claim 6, wherein the effective amount is 20 mg/day.

8. Tasimelteon for the use of claim 1, 2, 3, 4, 5, 6, or 7, wherein administering includes administering prior to bedtime.

9. Tasimelteon for the use of any of the preceding claims in which the patient is being treated with fluvoxamine, the treatment further comprising:
discontinuing treatment with fluvoxamine; and then
administering to the patient the effective amount of tasimelteon,
thereby avoiding the use of tasimelteon in combination with fluvoxamine.

10. Tasimelteon for the use of any of the preceding claims in which the patient is being treated with rifampicin, the treatment further comprising:
discontinuing treatment with rifampicin; and then
administering to the patient the effective amount of tasimelteon,
thereby avoiding the use of tasimelteon in combination with rifampicin.

11. Tasimelteon for the use of any of the preceding claims in which the patient smokes, the treatment further comprising:
discontinuing smoking by the patient; and then
administering to the patient the effective amount of tasimelteon,
thereby avoiding the use of tasimelteon in a patient who smokes.

12. Tasimelteon for the use of any of the preceding claims, wherein the administering includes administering to the patient the effective amount of tasimelteon without food.

## Patentansprüche

1. Tasimelteon zur Verwendung bei der Behandlung einer affektiven Störung bei mehrheitlich schwarzafrikanischen Patienten, wobei die Behandlung umfasst:
Verabreichen einer zur Behandlung der affektiven Störung wirksamen Menge Tasimelteon an den Patienten.

2. Tasimelteon zur Verwendung nach Anspruch 1, wobei es sich bei der affektiven Störung um eine depressive Störung handelt.

3. Tasimelteon zur Verwendung nach Anspruch 1, wobei der Patient mindestens ein Symptom aufweist, ausgewählt aus der Gruppe bestehend aus: Stimmung anhaltender Trauer, Angst oder Leere; Gefühlen der Hoffnungslosigkeit; Pessimismus; Gefühlen von Schuld, Wertlosigkeit oder Hilflosigkeit; Verlust des Interesses oder der Freude an Hobbys und Aktivitäten, die früher Spaß gemacht haben, einschließlich Sex; verminderter Energie, Erschöpfung oder Verlangsamung; Schwierigkeiten bei der Konzentration, beim Erinnern oder beim Treffen von Entscheidungen; Schlaflosigkeit, frühmorgendliches Erwachen oder Verschlafen; Appetit-und/oder Gewichtsverlust oder Esssucht und Gewichtszunahme; Gedanken an Tod oder Selbstmord; Selbstmordversuche; Unruhe; Reizbarkeit; anhaltende körperliche Symptome, die nicht auf eine Behandlung ansprechen, wie Kopfschmerzen, Verdauungsstörungen und chronische Schmerzen, oder irgendeine Kombination der vorstehend aufgeführten Symptome.

4. Tasimelteon zur Verwendung nach Anspruch 1, wobei es sich bei der affektiven Störung um eine schwere depressive Störung (Major Depressive Disorder, MDD) handelt.

5. Tasimelteon zur Verwendung nach Anspruch 1, 2, 3 oder 4, wobei die wirksame Menge zwischen 10 mg/Tag und 100 mg/Tag liegt.

6. Tasimelteon zur Verwendung nach Anspruch 5, wobei die wirksame Menge zwischen 20 mg/Tag und 50 mg/Tag liegt.

7. Tasimelteon zur Verwendung nach Anspruch 6, wobei die wirksame Menge 20 mg/Tag beträgt.

8. Tasimelteon zur Verwendung nach Anspruch 1, 2, 3, 4, 5, 6 oder 7, wobei das Verabreichen das Verabreichen vor dem Schlafengehen umfasst.

9. Tasimelteon zur Verwendung nach einem der vorstehenden Ansprüche, wobei der Patient mit Fluvoxamin behandelt wird, wobei die Behandlung ferner umfasst:
Absetzen der Behandlung mit Fluvoxamin und anschließend
Verabreichen der wirksamen Menge Tasimelteon an den Patienten,
dadurch Vermeiden der Verwendung von Tasimelteon in Kombination mit Fluvoxamin.

10. Tasimelteon zur Verwendung nach einem der vorstehenden Ansprüche, wobei der Patient mit Rifampicin behandelt wird, wobei die Behandlung ferner umfasst:
Absetzen der Behandlung mit Rifampicin und anschließend
Verabreichen der wirksamen Menge Tasimelteon an den Patienten,
dadurch Vermeiden der Verwendung von Tasimelteon in Kombination mit Rifampicin.

11. Tasimelteon zur Verwendung nach einem der vorstehenden Ansprüche, wobei es sich bei dem Patienten um einen Raucher handelt, wobei die Behandlung ferner umfasst:
Einstellen des Rauchens durch den Patienten und anschließend
Verabreichen der wirksamen Menge Tasimelteon an den Patienten,
dadurch Vermeiden der Verwendung von Tasimelteon bei einem Patienten, der raucht.

12. Tasimelteon zur Verwendung nach einem der vorstehenden Ansprüche, wobei das Verabreichen das Verabreichen der wirksamen Menge von Tasimelteon an den Patienten ohne Nahrungsaufnahme umfasst.

## Revendications

1. Tasimeltéon pour une utilisation dans le traitement d'un trouble affectif chez un patient d'une majorité noire africaine, le traitement comprenant :
une administration audit patient d'une quantité de tasimeltéon efficace pour traiter ledit trouble affectif.

2. Tasimeltéon pour l'utilisation selon la revendication 1, le trouble affectif étant un trouble dépressif.

3. Tasimeltéon pour l'utilisation selon la revendication 1, le patient présentant au moins un symptôme choisi dans un groupe constitué par : humeur triste, anxieuse ou vide persistante ; sentiments de désespoir ; pessimisme ; sentiments de culpabilité, d'inutilité ou d'impuissance ; perte d'intérêt ou de plaisir pour les passe-temps et les activités auparavant appréciés, y compris la sexualité ; baisse d'énergie, fatigue ou ralentissement ; difficulté à se concentrer, à se souvenir ou à prendre des décisions ; insomnie, réveil matinal ou sommeil excessif ; perte d'appétit et/ou de poids ou suralimentation et prise de poids ; pensées de mort ou de suicide ; tentatives de suicide ; agitation ; irritabilité ; symptômes physiques persistants qui ne répondent pas à un traitement, tels que maux de tête, troubles digestifs et douleurs chroniques ; ou toute combinaison de ce qui précède.

4. Tasimeltéon pour l'utilisation selon la revendication 1, le trouble affectif étant un trouble dépressif majeur (MDD).

5. Tasimeltéon pour l'utilisation selon la revendication 1, 2, 3 ou 4, la quantité efficace étant comprise entre 10 mg/jour et 100 mg/jour.

6. Tasimeltéon pour l'utilisation selon la revendication 5, la quantité efficace étant comprise entre 20 mg/jour et 50 mg/jour.

7. Tasimeltéon pour l'utilisation selon la revendication 6, la quantité efficace étant de 20 mg/jour.

8. Tasimeltéon pour l'utilisation selon la revendication 1, 2, 3, 4, 5, 6 ou 7, une administration comprenant une administration avant le coucher.

9. Tasimeltéon pour l'utilisation selon l'une quelconque des revendications précédentes dans laquelle le patient est traité avec de la fluvoxamine, le traitement comprenant en outre :
un arrêt d'un traitement avec de la fluvoxamine ; et ensuite
une administration au patient de la quantité efficace de tasimeltéon,
évitant ainsi l'utilisation de tasimeltéon en combinaison avec la fluvoxamine.

10. Tasimeltéon pour l'utilisation selon l'une quelconque des revendications précédentes dans laquelle le patient est traité avec de la rifampicine, le traitement comprenant en outre :
un arrêt d'un traitement avec de la rifampicine ; et ensuite
une administration au patient de la quantité efficace de tasimeltéon,
évitant ainsi l'utilisation de tasimeltéon en combinaison avec la rifampicine.

11. Tasimeltéon pour l'utilisation selon l'une quelconque des revendications précédentes dans laquelle le patient fume, le traitement comprenant en outre :
un arrêt du tabagisme par le patient ; et ensuite
une administration au patient de la quantité efficace de tasimeltéon,
évitant ainsi l'utilisation de tasimeltéon chez un patient qui fume.

12. Tasimeltéon pour l'utilisation selon l'une quelconque des revendications précédentes, l'administration comprenant une administration au patient de la quantité efficace de tasimeltéon sans aliment.
